# EUROPÄISCHE PATENTSCHRIFT

(11) **EP 3 903 682 B1**
(45) Veröffentlichungstag und Bekanntmachung des Hinweises auf die Patenterteilung: **04.10.2023**
(21) Anmeldenummer: 20172133.9
(22) Anmeldetag: 29.04.2020
(51) Int. Cl.: A61B 6/10

(54) **KOLLISIONSÜBERWACHUNG IN EINER MEDIZINISCHEN UMGEBUNG**
COLLISION MONITORING IN A MEDICAL ENVIRONMENT
SURVEILLANCE DE COLLISION DANS UN ENVIRONNEMENT MÉDICAL

(43) Veröffentlichungstag der Anmeldung: 03.11.2021
(73) Patentinhaber: Siemens Healthcare GmbH, 91052 Erlangen (DE)
(72) Erfinder: Garcia, Elmar, 90427 Nürnberg (DE)

(56) Entgegenhaltungen:
- US-A1- 2006 239 415
- US-A1- 2017 128 296
- US-A1- 2017 303 882
- US-B1- 6 950 025

## Beschreibung

Die Erfindung betrifft ein System zur Kollisionsüberwachung mit wenigstens einem ersten medizinischen Gerät und wenigstens einem Beschleunigungssensor, der ausgebildet ist, eine kollisionsbedingte Bewegung des ersten medizinischen Geräts zu detektieren.

Die Erfindung betrifft eine medizinische Umgebung wie bspw. ein Krankenhaus oder eine ambulante Klinik. Hier sind sich autonom, bzw. teil-autonom bewegende medizinische Geräte wie Patientenliegen oder fahrbare Assistenz- oder Bildgebungsgeräte auf dem Vormarsch. Zum Schutz von Patient, Personal und oft kostenintensiver Ausstattung ist für diese Geräte eine weitreichende Umgebungsüberwachungsfunktion unerlässlich, um ungewollte Kollisionen zu detektieren bzw. sogar zu vermeiden. Dazu werden die mobilen medizinischen Geräte mit verschiedenen Sensoren für die Umgebungserkennung ausgestattet, bspw. mit optischen, akustischen oder elektromagnetischen Sensoren. Insbesondere in beengten, nicht deterministischen Umgebungen wie klinischen Innenräumen, bspw. einem Operationssaal, einem Interventionsraum, einem Katheter-Labor, einem radiologischen Untersuchungsraum oder dergleichen kann es dennoch zu Kollisionen zwischen (teil-) autonom bewegten medizinischen Geräten und/oder Personal und insbesondere kleinen Objekten wie Infusions- und Gasschläuchen, Kabeln, Instrumentenhalterungen, Tischanbauten (z.B. Arm-, Beinhalter an OP-Tischen), Bleiabschirmwänden oder dergleichen kommen. Diese Objekte sind insbesondere durch eine verminderte optische Erfassbarkeit gekennzeichnet, sodass sie durch Personal als auch Sensorik nur sehr schwer detektierbar sind, bspw. weil sie vergleichsweise klein oder transparent sind. Beschädigungen an Gerät, Verletzung von Personen oder eine Störung eines medizinischen Arbeitsablaufes sind die Folge. Grund dafür ist, dass derartige kleine Objekte, im Folgenden auch medizinische Geräte genannt, nicht mittels der Umgebungsüberwachungsfunktion des mobilen medizinischen Geräts bzw. durch Personal jederzeit und vollumfänglich vollständig und zuverlässig erfasst und überwacht werden können.

Bislang müssen Kollisionen von (teil-)autonom bewegten medizinischen Geräten mit anderen medizinischen Geräten oder Gegenständen in Klinik-Innenräumen daher durch das Personal vorab ausgeschlossen werden. Die Verantwortung für die Gewährleistung kollisionsfreier Bewegungsräume wird somit dem Nutzer übertragen.

Die US 6,950,025 B1 offenbart eine Sicherheitsvorrichtung für die medizinische Chirurgie, die in Verbindung mit chirurgischen Instrumenten und anderen medizinischen Geräten im Operationssaal verwendet wird. Die Sicherheitsvorrichtung enthält eine oder mehrere Ferneinheiten, die an den chirurgischen Instrumenten und den anderen medizinischen Geräten angebracht sind und Beschleunigungsmesser zum Erfassen einer plötzlichen Bewegung der chirurgischen Instrumente und der anderen medizinischen Geräte und einen Hochfrequenzsignalsender zum Übertragen eines Hochfrequenzsignals bei Erfassung einer solchen plötzlichen Bewegung. Die Sicherheitsvorrichtung umfasst auch eine Basiseinheit, die an einem chirurgischen Hauptinstrument angebracht ist und einen Beschleunigungsmesser zum Erfassen einer plötzlichen Bewegung des chirurgischen Hauptinstruments und einen Hochfrequenzsignalempfänger zum Empfangen des Hochfrequenzsignals von den entfernten Einheiten. Die Basiseinheit umfasst auch einen Stromanschluss, der mit einer externen Stromquelle verbunden ist, um die chirurgischen Instrumente mit Strom zu versorgen. Die Stromzufuhr zu allen chirurgischen Instrumenten wird unterbrochen, wenn entweder der Beschleunigungsmesser der Basiseinheit eine plötzliche Bewegung feststellt oder wenn das Funkfrequenzsignal von den entfernten Einheiten empfangen wird.

Demgegenüber ist es Aufgabe der vorliegenden Erfindung, alternative Mittel bereit zu stellen, die es erlauben, zuverlässig und reproduzierbar Kollisionen eines medizinischen Geräts zu detektieren und der Kollision entgegen zu wirken. Insbesondere ist es Aufgabe der vorliegenden Erfindung, Kollisionen derjenigen medizinischen Geräte zu erfassen, die mittels klassischer Sensortechnik nicht zuverlässig überwacht werden können.

Diese Aufgabe wird gelöst durch ein medizinisches System zur Kollisionsüberwachung gemäß Anspruch 1. Bevorzugte und/oder alternative, vorteilhafte Ausgestaltungsvarianten sind Gegenstand der abhängigen Ansprüche.

Nachstehend wird die erfindungsgemäße Lösung der Aufgabe in Bezug auf die beanspruchte Vorrichtung beschrieben. Hierbei erwähnte Merkmale, Vorteile oder alternative Ausführungsformen sind, auch wenn sie beispielsweise auf ein Verfahren gerichtet sind, auch mit Merkmalen, die in Zusammenhang mit der beanspruchten Vorrichtung beschrieben oder beansprucht sind, kombinierbar. Die entsprechenden funktionalen Verfahrensmerkmale können dabei durch entsprechende gegenständliche Module oder Einheiten der beanspruchten Vorrichtung ausgebildet werden.

Das erfindungsgemäße medizinische System ist für eine Kollisionsüberwachung in einer medizinischen Umgebung ausgebildet.

Es umfasst wenigstens ein erstes medizinisches Gerät und wenigstens einen Beschleunigungssensor. Das erste medizinische Gerät nimmt zur Ausführung eines medizinischen Arbeitsablaufes eine Zielposition in der medizinischen Umgebung ein. Der Beschleunigungssensor ist an dem ersten medizinischen Gerät angeordnet und ausgebildet, bei kollisionsbedingter Bewegung des ersten medizinischen Geräts aus seiner Zielposition heraus eine Beschleunigung zu erfassen und ein Warnsignal an die medizinische Umgebung auszugeben.

Das erfindungsgemäße medizinische System umfasst mindestens eine medizinische Einheit, bevorzugt aber eine Mehrzahl an medizinischen Einheiten. Diese können als medizinisches Gerät ausgebildet sein. Ein medizinisches Gerät kann einer medizinischen Bildgebungsanlage oder einzelnen Komponenten davon entsprechen. Bspw. kann ein medizinisches Gerät als Ultraschall- oder Röntgengerät, Computertomographie-Gerät oder dergleichen ausgebildet sein. Das medizinische Gerät kann auch nur eine Einheit umfassend eine Komponente der medizinischen Bildgebung, bspw. Röntgenstrahlungsquelle oder Röntgendetektor sein. Das medizinische Gerät kann auch ein medizinisches Assistenzgerät oder eine Komponente davon sein, bspw. ein Gerät zur Überwachung physiologischer Funktionen eines Patienten, bspw. ein EKG-Gerät, oder ein Beatmungsgerät, ein Infusionsständer mit Infusionsschlauch, ein Beatmungsgerät mit Beatmungsschlauch und Maske, ein Instrumenten- oder Katheter-Halter, ein Monitor, ein medizinsicher Raumteiler, ein Strahlenschutz oder ein Sichtschutz oder dergleichen sein.

Ein medizinisches Gerät kann auch als Patiententisch oder Patientenliege ausgebildet sein, oder einer Komponente davon entsprechen, bspw. einem Tischanbau oder einem Extremitäten-Halter.

Das medizinische System ist in einer medizinischen Umgebung angeordnet. Typischerweise handelt es sich dabei um einen Untersuchungsraum, einen OP-Saal oder ein Krankenzimmer in einem Krankenhaus oder einer ambulanten Einrichtung oder einer Arztpraxis. Die medizinische Umgebung kann auch mehrere Räume oder ein ganzes Gebäude umfassen.

Das wenigstens eine erste medizinische Gerät des erfindungsgemäßen Systems ist bevorzugt als vergleichsweise kleines, medizinisches Gerät oder als kleine Komponente desselben ausgebildet. Insbesondere handelt es sich bei dem ersten medizinischen Gerät um einen Schlauch, ein Kabel, einen Halter, einen Ständer, einen Katheter, ein Instrument, einen Monitor oder dergleichen. Alternativ ist das erste medizinische Gerät bevorzugt als farbloses bzw. transparentes medizinisches Gerät ausgebildet, bspw. als Strahlen-, Sicht- oder Spritzschutz. Es kann insbesondere aus Bleiglas oder Plexiglas bestehen. Das erste medizinische Gerät ist dadurch gekennzeichnet, dass es nicht gut sichtbar bzw. schwer optisch bzw. automatisch erfassbar ist.

Das erste medizinische Gerät ist grundsätzlich ein bewegliches, mobiles medizinisches Gerät oder eine Komponente oder ein (Bestand-)Teil. Für einen medizinischen Arbeitsablauf, einen sogenannten Workflow, bspw. eine medizinische Bildaufnahmeprozedur und/oder ein medizinischer Eingriff, befindet sich das erste medizinische Gerät in einer Zielposition. Mit anderen Worten, das erste medizinische Gerät ist während der Dauer eines Arbeitsablaufes in seiner Zielposition angeordnet und bewegt sich währenddessen nicht selbständig aus der Zielposition heraus. Bspw. ist ein Extremitäten-Halter für einen medizinischen Eingriff an einem Patiententisch in einer vorab definierten Position, der Zielposition fixiert, kann aber entfernt werden. Gleiches gilt bspw. für einen verstellbaren Strahlenschutz oder andere erste medizinische Geräte. In Ausführungen kann das erste medizinische Gerät als (teil- )autonomes medizinisches Gerät ausgebildet sein und sich nach Abschluss des medizinischen Arbeitsablaufs selber aus der Zielposition entfernen/verstellen. In anderen Ausführungen ist das erste medizinische Gerät unbeweglich ausgebildet, sodass es bspw. nur passiv durch einen Nutzer verstellt werden kann.

Es ist wenigstens ein erstes medizinisches Gerät vorgesehen. Mit anderen Worten kann das erfindungsgemäße System eine Vielzahl von ersten medizinischen Geräten umfassen.

Der vom System umfasste Beschleunigungssensor ist an dem ersten medizinischen Gerät, insbesondere an einer seiner Komponenten, angeordnet. Besonders bevorzugt ist der Beschleunigungssensor an einer Außenseite des ersten medizinischen Geräts angeordnet. Der Beschleunigungssensor ist ausgebildet eine kollisionsbedingte Bewegung des ersten medizinischen Geräts aus der Zielposition heraus zu erfassen. Mit anderen Worten wird das erste medizinische Gerät aus der Zielposition gebracht, detektiert bzw. misst der Beschleunigungssensor die durch die Bewegung verursachte Beschleunigung, die auf das erste medizinische Gerät wirkt.

Die Bewegung des ersten medizinischen Geräts aus der Zielposition heraus erfolgt kollisionsbedingt, d.h. infolge einer Berührung/eines Aufpralls/eines Zusammenstoßes eines weiteren medizinischen Geräts oder einer Person, bspw. medizinischem Personal oder dem Patienten mit dem ersten medizinischen Gerät. Die Berührung/der Aufprall/der Zusammenstoß erfolgen durch Bewegung des Weiteren medizinischen Geräts oder der Person.

Der Beschleunigungssensor kann in Ausführungen der Erfindung ausgebildet sein, einen Wert für eine auf das erste medizinische Gerät wirkende Beschleunigung zu erfassen. Alternativ kann der Beschleunigungssensor ausgebildet sein, zu erkennen, wenn bzw. ob eine Beschleunigung anliegt, ohne den konkreten Wert der Beschleunigung zu ermitteln. Der Beschleunigungssensor kann mehrere Betriebsmodi umfassen, die wobei jeweils ein Betriebsmodus eine der oben beschriebenen Varianten realisiert. Der Beschleunigungssensor kann ausgebildet sein, dass zwischen den Betriebsmodi gewechselt werden kann.

Der Beschleunigungssensor kann bevorzugt eine Piezoelektrische oder mikro-elektro-mechanische (MEMS) Detektionseinheit umfassen. Deren Wirkprinzipien sind jeweils an sich bekannt. Beschleunigungssensoren sind sehr kostengünstig. Insbesondere ein MEMS-Beschleunigungssensor kann zudem in Miniaturausführung realisiert werden, wodurch der medizinische Arbeitsablauf oder die Funktion des ersten medizinischen Geräts nicht beeinträchtigt wird.

Der Beschleunigungssensor ist weiter ausgebildet, bei Detektion einer auf das erste medizinische Gerät wirkenden Beschleunigung ein Warnsignal an die medizinische Umgebung auszugeben. Mit anderen Worten umfasst der Beschleunigungssensor eine Ausgabeeinheit, die ausgebildet ist, das Warnsignal insbesondere basierend auf dem Wert einer erfassten Beschleunigung zu erzeugen und auszugeben. Das Warnsignal dient dazu, in der medizinischen Umgebung befindliches medizinisches Personal auf eine aktuelle Kollision aufmerksam zu machen. Zusätzlich kann das Warnsignal derart ausgebildet sein, dass ein weiteres medizinisches Gerät anhand des Warnsignals das erste medizinische Gerät in der medizinischen Umgebung bzw. eine Kollisionssituation erkennt.

Der Erfinder hat erkannt, dass insbesondere kleine Objekte in Form von medizinischen Geräten oder deren Komponenten, welche mit optischen, akustischen oder elektromagnetischen Sensoren nur schwer bis gar nicht messtechnisch zuverlässig erfassbar sind, zumindest dann bei einer Kollisionsüberwachung berücksichtigt werden können, wenn infolge ihrer kollisionsbedingten Bewegung bzw. Erschütterung ein Warnsignal erzeugt wird.

Der Erfindung liegt also die Erkenntnis zugrunde, ein erstes medizinisches Gerät, das während eines medizinischen Arbeitsablaufs an sich unbewegt ist, für weitere bewegte medizinische Geräte und anwesende Personen erkennbar bzw. detektierbar zu machen, indem man auch das erste medizinische Gerät mit Sensorik in Form eines Beschleunigungssensors ausstattet. Dies erübrigt vorteilhaft ein weiteres Ausrüsten von vom System ebenfalls umfassten, bewegten medizinischen Geräten mit weiteren, teilweise aufwändigen oder teuren Sensorsystemen zur Umgebungsüberwachung. Erfindungsgemäß werden genau diejenigen medizinischen Geräte mit Beschleunigungssensoren versehen, bei deren eine ungewollte Kollision besonders nachteilig wäre.

In einer ersten Ausführung der Erfindung ist der Beschleunigungssensor ausgebildet, eine Beschleunigung in einer beliebigen Raumrichtung zu erfassen. Mit anderen Worten ist der Beschleunigungssensor sensibel für Kollisionen in jeder Raumrichtung. In Ausführungen der Erfindung verfügt der Beschleunigungssensor über ein drei-dimensionales Referenzkoordinatensystem mit drei orthogonal zu einander angeordneten Raumachsen, wobei eine gerichtete Beschleunigung entlang eines beliebigen Richtungsvektors in dem Referenzkoordinatensystem detektiert werden kann. Dies macht die Erfindung besonders flexibel einsetzbar.

Gemäß der Erfindung ist der Beschleunigungssensor ausgebildet, ein optisches und/oder akustisches Warnsignal auszugeben. Zu diesem Zweck umfasst der Beschleunigungssensor eine Ausgabe-Einheit, die ausgebildet ist, ein optisches und/oder akustisches Warnsignal zu erzeugen und auszugeben. Bspw. umfasst der Beschleunigungssensor ein Leuchtmittel in Form einer LED und/oder einen Lautsprecher, die ausgebildet sind, das Warnsignal auszugeben. Besonders bevorzugt ist das Leuchtmittel derart an einer Außenseite des Beschleunigungssensors angeordnet, dass es durch einen Benutzer, medizinisches Personal, gut wahrgenommen bzw. erkannt werden kann.

In einer weiteren Ausführung der Erfindung ist der Beschleunigungssensor ausgebildet, einen erfassten Wert der Beschleunigung mit einem vorab definierten Schwellwert zu vergleichen und nur bei Überschreiten des Schwellwerts ein Warnsignal auszugeben. Der vorab definierte Schwellwert für die Beschleunigung kann in Ausführungen kennzeichnend sein für eine maximal zulässige Beschleunigung, bei der keine Beschädigungen/Verletzungen zu erwarten sind. Dieser Schwellwert kann zuvor empirisch ermittelt werden. Dieser Schwellwert kann insbesondere von der Ausführung bzw. der Art des ersten medizinischen Geräts abhängen und entsprechend variieren. Der Schwellwert kann am Beschleunigungssensor werksseitig voreingestellt oder nachträglich anpassbar sein. In dieser Ausführung ist der Beschleunigungssensor ausgebildet, den erfassten Wert mit dem vorab definierten Schwellwert in Bezug zu setzen. Eine Ausgabe eines Warnsignals erfolgt nur dann, wenn der Schwellwert von dem erfassten Wert der Beschleunigung am ersten medizinischen Gerät überschritten wird. Bei Unterschreiten des Schwellwerts erkennt der Beschleunigungssensor also die kollisionsbedingte Bewegung vorteilhaft als unkritische Bewegung des ersten medizinischen Geräts, bei welcher kein Warnsignal ausgegeben wird. Derart stellt die Erfindung sicher, dass nur bei heftigen/starken und daher kritischen (Zusammen-)Stößen ein Warnsignal ausgegeben wird, sodass in allen anderen Fällen der medizinische Arbeitsablauf nicht gestört oder unterbrochen werden braucht.

In einer weiteren Ausführung der Erfindung ist der Beschleunigungssensor ausgebildet, eine Signalstärke des Warnsignals an einen erfassten Wert der Beschleunigung anzupassen. Bei dieser Ausführung wird also bspw. die Helligkeit des Leuchtmittels oder die Lautstärke am Lautsprecher in Abhängigkeit des erfassten Wertes der Beschleunigung angepasst. Mit anderen Worten, das Leuchtmittel gibt ein umso helleres Warnsignal aus, je größer der Wert der Beschleunigung und damit je heftiger der Aufprall ist und ein umso dunkleres, wenn lediglich ein geringer Beschleunigungswert erfasst wurde. Gleichsam kann es sich mit der Lautstärke eines Warnsignals verhalten, welche umso lauter ist, je größer der erfasste Beschleunigungswert ist. Hierzu kann in einer Speichervorrichtung des Beschleunigungssensors eine Look-Up Tabelle zum Abruf hinterlegt sein, die bspw. Wertebereichen der Beschleunigung jeweils einen Intensitäts- oder Lautstärkewert zuordnet. Diese Ausführung bewirkt vorteilhaft, dass durch die insbesondere bei starken Kollisionen erhöhte Warnsignalintensität leichter bzw. schneller durch Personal erkannt werden und Gegenmaßnahmen ergriffen werden können.

In einer weiteren Ausführung der Erfindung umfasst der Beschleunigungssensor einen Schnellverschluss. Der Schnellverschluss dient vorteilhaft einer einfachen und schnellen Anbringung des Beschleunigungssensors an dem ersten medizinischen Gerät. Der Schnellverschluss ist besonders vorteilhaft an der Außenseite des Beschleunigungssensors angebracht bzw. kann einstückig mit dieser ausgebildet sein. Der Schnellverschluss kann als Einweg- oder wiederverwendbarer Verschluss ausgebildet sein. In letztem Fall kann der Beschleunigungssensor an mehreren ersten medizinischen Geräten nach einander zum Einsatz kommen oder vereinfacht umpositioniert werden, wo erforderlich.

In einer weiteren, besonders bevorzugten Ausführung der Erfindung ist der Schnellverschluss daher lösbar und wiederverschließbar ausgebildet. Insbesondere ist der Schnellverschluss auch lösbar von dem Beschleunigungssensor ausgebildet, sodass nicht nur der Beschleunigungssensor samt Schnellverschluss wiedereinsetzbar ist, sondern auch der Schnellverschluss an sich an mehreren Beschleunigungssensoren verwendet werden kann. Durch den lösbaren und wiederverschließbaren Schnellverschluss kann der Beschleunigungssensor vorteilhaft an dem ersten medizinischen Gerät umpositioniert werden, bspw. nach unkorrekter Erstplatzierung. Derart kann Ressourcen-schonend und kostengünstig agiert werden. Der Schnellverschluss kann insbesondere als Klettverschluss, als Riemenverschluss, als Schnallenverschluss, als lösbarer Steckverschluss oder dergleichen ausgebildet sein. Insbesondere ist der Schnellverschluss längenverstellbar ausgebildet, sodass der Beschleunigungssensor an beliebig ausgeformten ersten medizinischen Geräten angebracht werden kann.

In einer weiteren Ausführung der Erfindung umfasst das medizinische System eine Vielzahl von ersten medizinischen Geräten, wobei an jedem ersten medizinischen Gerät ein Beschleunigungssensor angeordnet ist. Das erfindungsgemäße System trägt in dieser Ausführung der Tatsache Rechnung, dass eine medizinische Umgebung mehrere, also nicht nur ein erstes medizinisches Gerät umfasst, welches optisch oder anders sensorisch nur schwer erfassbar ist. Die Funktionssicherheit aller medizinischen Geräte und die Sicherheit von Personal und Patient in der medizinischen Umgebung wird vorteilhaft maximiert, wenn jedes der ersten medizinischen Geräte mit einem erfindungsgemäßen Beschleunigungssensor ausgestattet ist, der jeweils bei Kollision ein Warnsignal ausgibt.

Gemäß der Erfindung umfasst das medizinische System ein zweites medizinisches Gerät, welches ausgebildet ist, in der medizinischen Umgebung eine teilautonome oder autonome Bewegung auszuführen. Das zweite medizinische Gerät ist also dadurch gekennzeichnet, dass es sich in der medizinischen Umgebung bewegt, und insbesondere während des medizinischen Arbeitsablaufes bewegt. Die Bewegung kann durch einen Bediener geführt oder autorisiert erfolgen. Die Bewegung kann auch selbständig, also autonom, also ohne Benutzerinterkation erfolgen. Insbesondere im letzten Fall kommen die erfindungsgemäßen Vorteile zum Tragen, da dort keine menschliche Kontrolle für die Bewegung des zweiten medizinischen Gerätes mehr vorgesehen ist. Das zweite medizinische Gerät kann bspw. als Patientenliege, als mobile Bildgebungsanlage, bspw. ein mobiles Ultraschall- oder EKG-Gerät oder eine CT-Gantry oder dergleichen ausgebildet sein. Das zweite medizinische Gerät kann auch als medizinische Assistenz- oder Versorgungs-Einheit (Instrumente/medizinisches Material, etc.) oder als Assistenz-Roboter ausgebildet sein.

In einer weiteren Ausführung kann das medizinische System auch mehrere, also wenigstens zwei, zweite medizinische Geräte umfassen, die sich zumindest teilautonom in der Krankenhausumgebung bewegen können.

Gemäß der Erfindung ist das zweite medizinische Gerät ausgebildet, das optische und/oder akustische Warnsignal des wenigstens einen ersten Beschleunigungssensors zu erfassen und seine teilautonome oder autonome Bewegung zu stoppen. In dieser Ausführung verfügt also das zweite medizinische Gerät über wenigstens eine Sensoreinheit, die ausgebildet ist, das vom Beschleunigungssensor ausgegebene Warnsignal zu erfassen. Die Sensoreinheit kann einen akustischen, optischen oder andersartigen Sensor umfassen, also bspw. eine Kamera oder ein Mikrophon. Eine Kombination mehrerer Sensoren in der Sensoreinheit macht die Erfindung vorteilhaft unabhängig von dem zum Einsatz kommenden Beschleunigungssensor. Derart können insbesondere auch verschiedene Warnsignale verschiedener Beschleunigungssensoren erfasst werden.

In dieser Ausführung umfasst das zweite medizinische Gerät auch eine Auswerte- bzw. Recheneinheit, die mit der Sensoreinheit in Datenaustausch steht. Die Auswerteeinheit ist ausgebildet, Steuersignale für eine Bewegung, insbesondere eine teilautonome Bewegung des zweiten medizinischen Geräts, bspw. für sein Fahrwerk zu erzeugen. Ein Steuersignal kann dabei bspw. eine Bewegungstrajektorie in der medizinischen Umgebung oder eine Bewegungsgeschwindigkeit definieren. Die Auswerteeinheit ist in dieser Ausführung weiter ausgebildet, ein erfasstes Warnsignal zu verwenden, um basierend darauf wenigstens ein Steuersignal, bspw. für das Fahrwerk anzupassen bzw. zu erzeugen. Bspw. kann die Auswerteeinheit basierend auf dem kollisionsbedingten Warnsignal ein Steuersignal mit einem ggü. einem zuvor festgelegten Geschwindigkeitswert reduzierten Geschwindigkeit oder zum Stoppen oder für eine Richtungsumkehr einer autonomen Bewegung des zweiten medizinischen Geräts erzeugen. Die Auswerteeinheit kann ausgebildet sein, das zu erzeugende Steuersignal bspw. an den erfassten Wert der Beschleunigung anzupassen.

In einer weiteren Ausführung der Erfindung stehen der wenigstens eine Beschleunigungssensor und das zweite medizinische Gerät über einen Datenkommunikationspfad direkt miteinander in Kontakt. Ferner ist der Beschleunigungssensor ausgebildet, ein Beschleunigungssignal an das zweite medizinische Gerät zu senden und das zweite medizinische Gerät ist ausgebildet, das Beschleunigungssignal zu erfassen. Alternativ oder zusätzlich können also die Auswerteeinheit des zweiten medizinischen Geräts und der Beschleunigungssensor direkt die erfassten Warnsignal-Daten mit einander austauschen. Dadurch kann vorteilhaft eine Detektion des Warnsignals mittels Sensoreinheit des zweiten medizinischen Geräts entfallen. Dies ist einerseits Ressourcen-schonend, zum anderen wird derart das Erkennen einer Kollisionssituation an dem zweiten medizinischen Gerät sichergestellt.

Ein Datenaustausch erfolgt bevorzugt mittels einer Netzwerk-Verbindung. Das Netzwerk kann als local area network (LAN), bspw. Ein Intranet oder ein wide area network (WAN) ausgebildet sein. Die Netzwerkverbindung ist bevorzugt kabellos ausgebildet, bspw. als wireless LAN (WLAN oder WiFi). Das Netzwerk kann eine Kombination aus verschiedenen Netzwerkbeispielen umfassen. In Ausführungen kann der Datenaustausch auch über Nahfeldkommunikation, bspw. Bluetooth erfolgen.

Das Konzept der vorliegenden Erfindung ist es also zusammengefasst, weitere Einheiten der medizinischen Umgebung, also erste medizinischen Geräte, mit Sensorik auszustatten und sie dadurch "intelligenter" zu machen, anstatt bewegliche, und insbesondere autonom bewegliche, also zweite medizinische Geräte mit zusätzlicher Sensorik auszustatten um die Umgebung zu erkennen/überwachen. Konkret besteht die Erfindung in der Anbringung kostengünstiger, wiederverwendbarer Beschleunigungssensoren an externes, schlecht optisch oder anders detektierbares Equipment, um eine Kollisionserkennung zu gewährleisten.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden. Durch diese Beschreibung erfolgt keine Beschränkung der Erfindung auf diese Ausführungsbeispiele. In verschiedenen Figuren sind gleiche Komponenten mit identischen Bezugszeichen versehen. Die Figuren sind in der Regel nicht maßstäblich. Es zeigen:
- FIG 1: eine Ansicht eines medizinischen Systems umfassend zwei Beschleunigungssensoren in einer Ausführungsform der vorliegenden Erfindung,
- FIG 2: eine Darstellung eines Beschleunigungssensors in einer Ausführungsform der vorliegenden Erfindung, und
- FIG 3, 4, und 5: alternative Ausführungsvarianten eines erfindungsgemäßen Beschleunigungssensors.

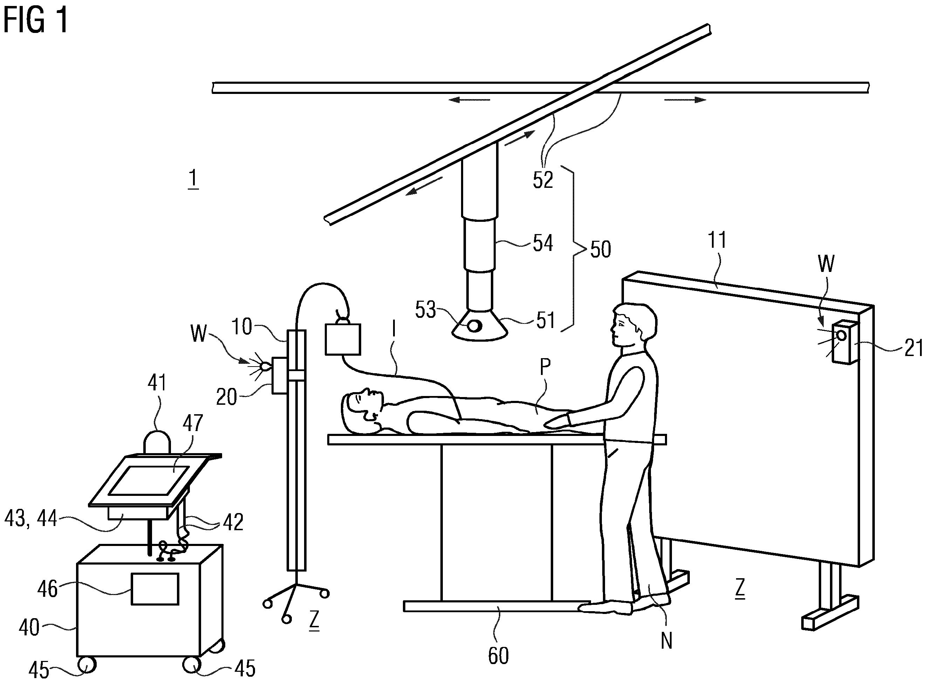

**Figur 1** zeigt eine Ansicht eines medizinischen Systems 1 umfassend zwei Beschleunigungssensoren 20, 21 in einer Ausführungsform der vorliegenden Erfindung. Das medizinische System dient der Kollisionsüberwachung in einer medizinischen Umgebung, hier einem medizinischen Untersuchungs- bzw. Behandlungsraum. Dieser kann sich in einer Klinik, einer Arztpraxis oder dergleichen befinden. Im Zentrum der medizinischen Umgebung/des Behandlungsraums befindet sich eine Patientenlagerungsvorrichtung in Form eines Untersuchungstisches 60, auf dem ein Patient P liegend positioniert ist. Die Behandlung/Untersuchung am Patienten P wird durch einen Nutzer N, medizinisches Personal oder einen Arzt, durchgeführt. Das hier gezeigte medizinische System umfasst eine Vielzahl, also zwei, von ersten medizinischen Geräten 10, 11, wobei an jedem ersten medizinischen Gerät ein Beschleunigungssensor 20, 21 angeordnet ist. Alternativ könnte auch lediglich ein erstes medizinisches Gerät 10 oder weitere erste medizinische Geräte vorgesehen sein. Die gezeigten ersten medizinischen Geräte 10, 11 sind als Infusionsständer und Strahlenschutzwand ausgebildet. Beiden ersten medizinischen Geräten 10, 11 ist gemein, dass sie nicht stationär, also grundsätzlich verfahrbar ausgebildet sind. Jedoch sind sie für einen medizinischen Arbeitsablauf jeweils in einer Zielposition Z angeordnet, welche einer gewünschten Position entspricht. Der Infusionsständer 10 ist nahe am Patienten P angeordnet, da er mittels Infusionsschlauch I diesen mit der Infusion, bspw. ein Kontrastmittel, versorgt. Die Strahlenschutzwand 11, befindet sich in ihrer Zielposition Z, in welcher sie dahinter liegende Bereiche des Untersuchungsraumes und insbesondere weiteres medizinisches Personal vor Röntgenstrahlung schützt. Während des medizinischen Arbeitsablaufes sollen die ersten medizinischen Geräte in ihren Zielpositionen verbleiben. Der Infusionsständer 10 und die Strahlenschutzwand 11 sind entweder schmal oder durchsichtig, sodass sie schwer sichtbar bzw.

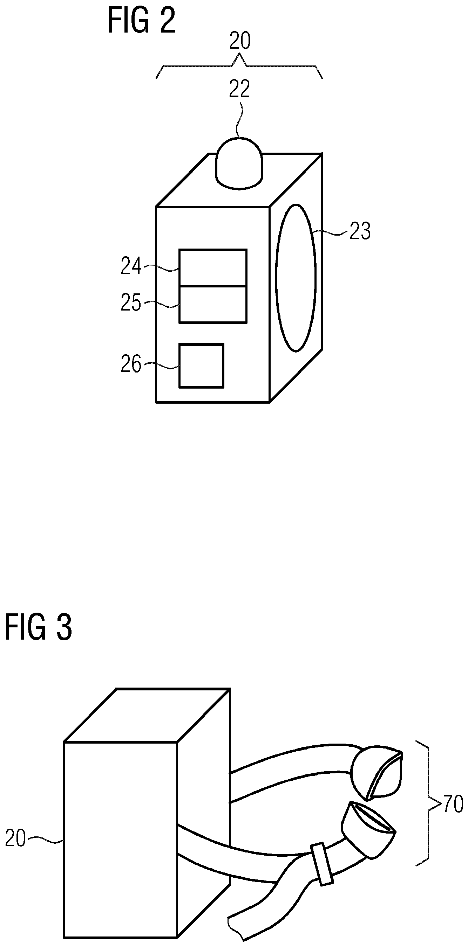

sensorisch erfassbar sind. Die Erfassbarkeit wird dadurch erschwert, dass die jeweiligen Zielpositionen Z von medizinischem Arbeitsablauf bzw. Patient abhängen. Die ersten medizinischen Geräte 10, 11 weisen jeweils einen Beschleunigungssensor 20, 21 auf. Die Beschleunigungssensoren 20, 21 sind jeweils an dem ersten medizinischen Gerät 10, 11 angeordnet. Sie sind ausgebildet, bei kollisionsbedingter Bewegung eines der ersten medizinischen Geräte 10, 11 aus seiner Zielposition heraus eine Beschleunigung zu erfassen und ein Warnsignal W an die medizinische Umgebung auszugeben. Die Ausgestaltung eines Beschleunigungssensors wird mit Bezug zu den Figuren 2 bis 5 weiter unten im Detail beschrieben.

Eine kollisionsbedingte Beschleunigung ist eine unabsichtliche Beschleunigung. Sie kann bspw. durch einen unvorhergesehenen Aufprall eines zweiten medizinischen Geräts 40, 50 oder dem Nutzer N mit einem der ersten medizinischen Geräte erfolgen, wenn sich die zweiten medizinischen Geräte 40, 50 oder der Nutzer N bewegen. Während die ersten medizinischen Geräte 10, 11 nur passiv bewegbar sind, können die zweiten medizinischen Geräte aktiv und insbesondere (teil-)autonom während des medizinischen Arbeitsablaufes im Untersuchungsraum verfahren. Das mobile EKG-Messgerät 40 umfasst zu diesem Zweck ein Fahrwerk 45 umfassend vier Räder, sowie eine Steuereinheit 46, um Steuersignale, bspw. Bewegungstrajektorien oder Zielpositionen zu ermitteln und Steuersignale für das Fahrwerk 45 zu erzeugen. Das mobile EKG-Gerät 40 umfasst über Kabel 42 angebundene EKG-Elektroden und einen Anzeige-Monitor 47 zur Anzeige des EKG-Signals für den Nutzer. Das mobile Deckenstativ 50 umfassend einen endständig angeordneten Röntgenstrahler 51 kann im Untersuchungsraum entlang eines Decken-gehängten Schienensystems 52 horizontal und mittels eines Teleskop-Arms 54 vertikal verstellt werden. Nun kann es zwischen zweiten medizinischen Geräten 40, 50 sowie dem Nutzer N und einem der ersten medizinischen Geräte 10, 11 unbeabsichtigt zu einem Zusammenstoß kommen, wenn sich erstere im Untersuchungsraum bewegen.

Ein Zusammenstoß verursacht das Erfassen eines Beschleunigungswertes mit einem der Beschleunigungssensoren 20, 21. Ein Warnsignal W wird dann basierend auf dem erfassten Beschleunigungswert mittels einer der beiden oder beiden Ausgabeeinheiten 22, 23 erzeugt und für die Umwelt ausgegeben. Das Warnsignal kann durch den Nutzer N optisch oder akustisch erfassbar sein. Alternativ kann das Warnsignal auch für die zweiten medizinischen Geräte erfassbar sein. Entsprechend umfassen die zweiten medizinischen Geräte 40, 50 Überwachungseinheiten in Form einer optischen (sichtbares Licht) Kamera 41 und eines Mikrophons 53. Sofern das Warnsignal mittels Überwachungseinheit 41, 53 erfasst wird, hat auch das zweite medizinische Gerät 40, 50 Kenntnis von der Kollision und kann reagieren. Das Warnsignal W ist entsprechend bevorzugt als optisches und/oder akustisches Warnsignal W ausgebildet.

**Figur 2** zeigt eine Darstellung eines Beschleunigungssensors 20 in einer Ausführungsform der vorliegenden Erfindung. Der hier gezeigte Beschleunigungssensor 20 umfasst eine Detektionseinheit 24, hier ein MEMS-Sensor. Dieser ist ausgebildet, auf ihn wirkende Beschleunigungen zu detektieren. Bevorzugt werden Beschleunigungen erkannt, die in der Horizontalebene wirken, aber grundsätzlich können Beschleunigungen in allen Raumrichtungen erfasst werden.

Der Beschleunigungssensor 20 umfasst in dieser Ausführung auch eine Auswerte-Einheit 25. Diese ist ausgebildet, einen erfassten Beschleunigungswert von der Detektionseinheit 24 zu empfangen und weiter zu verarbeiten. Die Auswerte-Einheit 25 ist ausgebildet, basierend auf dem empfangenen Beschleunigungswert wenigstens ein Steuersignal zur Erzeugung eines Warnsignals W zu erzeugen. Dazu kann die Auswerteeinheit 25 ausgebildet sein, lediglich zu prüfen, ob ein Beschleunigungswert vorliegt oder nicht und falls ja, ein Steuersignal für eine Standard-Warnsignal W zu erzeugen. Alternativ dazu, kann die Auswerteinheit 25 ausgebildet sein, einen erfassten Beschleunigungswert mit einem vorab definierten Schwellwert zu vergleichen und nur bei Überschreiten des Schwellwerts ein Steuersignal zur Erzeugung eines Warnsignals W zu erzeugen. Hierbei besteht die Möglichkeit, das Steuersignal für die Erzeugung des Standard-Warnsignals W oder das Steuersignal für ein an den erfassten Beschleunigungswert angepassten Warnsignals zu erzeugen. Bspw. kann die Signalstärke, bspw. die Helligkeit bzw. die Lautstärke des Warnsignals W von dem erfassten Beschleunigungswert abhängen und jeweils mit diesem steigen.

Der hier gezeigte Beschleunigungssensor umfasst ferner Ausgabeeinheiten 22 und 23 in Form einer LED-Lampe und eines Lautsprechers. Andere oder nur eine der Ausgabeeinheiten 22, 23 sind ebenfalls möglich. Die Kombination mehrerer verschiedener Ausgabeeinheiten macht den Beschleunigungssensor 20 flexibel für verschiedene Benutzungssituationen einsetzbar. Die Ausgabeeinheiten 22, 23 dienen dem Zweck, infolge einer Kollision des ersten medizinischen Geräts 10 ein Warnsignal W zu erzeugen und auszugeben.

Die Auswerteinheit 25 kann in Form von Hard- oder in Form von Software ausgebildet sein. Beispielsweise ist die Auswerteeinheit 25 als ein sogenanntes FPGA (Akronym für das englischsprachige "Field Programmable Gate Array") ausgebildet oder umfasst eine arithmetische Logikeinheit. Die Auswerteeinheit 25 kann, so wie hier dargestellt, als Subkomponente des Beschleunigungssensors 20 oder auch als (eigenständiger) Cloud-basierter Computer ausgebildet sein, wobei der Datenaustausch mit dem Beschleunigungssensor 20 über eine sichere Internet-Verbindung erfolgt.

Detektionseinheit 24, Auswerteinheit 25 und die Ausgabeeinheiten 22, 23 stehen für einen Datenaustausch, bspw. erfasster Beschleunigungswerte oder erzeugter Steuersignale, zumindest unidirektional über Kommunikationsschnittstellen in Verbindung.

Neben oder alternativ zu wenigstens einer Ausgabeeinheit 22, 23 am Beschleunigungssensor 20 kann eine Schnittstelle 26 vorgesehen sein, über die eine Datenkommunikation mit wenigstens einem zweiten medizinischen Gerät 40 erfolgen kann. Über diese Schnittstelle können Informationen in Form eines digitalen Warnsignals über eine detektierte Kollision direkt mit einem zweiten medizinischen Gerät 40, insbesondere dem Kollisionspartner, ausgetauscht werden. Derart wird das zweite medizinische Gerät direkt über die Kollision informiert und kann Gegenmaßnahmen einleiten, bspw. eine Bewegung stoppen, verlangsamen oder eine Bewegungsrichtung ändern. Das zweite medizinische Gerät 40 verfügt zu diesem Zweck ebenfalls über eine Schnittstelle 43 und eine Auswerte-Einheit 44. Diese ist ausgebildet, das empfangene digitale Warnsignal zu verarbeiten und basierend darauf Steuersignale für das zweite medizinische Gerät 40, insbesondere sein Fahrwerk 45 zu erzeugen.

Die Schnittstelle 26 wird bspw. über eine Hardware- oder Software-Schnittstelle wie PCI-Bus, USB oder Firewire realisiert. Ein Datenaustausch erfolgt bevorzugt mittels einer Netzwerk-Verbindung. Das Netzwerk kann als local area network (LAN), bspw. Ein Intranet oder ein wide area network (WAN) ausgebildet sein. Die Netzwerkverbindung ist erfindungsgemäß kabellos ausgebildet, bspw. als wireless LAN (WLAN oder WiFi). Das Netzwerk kann eine Kombination aus verschiedenen Netzwerkbeispielen umfassen. Datenübertragung kann basierend auf einer Datenabfrage oder eigeninitiativ erfolgen. Datenübertragung zwischen zwei Einheiten bzw. Systemkomponenten kann bidirektional oder unidirektional erfolgen.

Die **Figuren 3 bis 5** zeigen jeweils alternative Ausführungsvarianten eines erfindungsgemäßen Beschleunigungssensors 20.

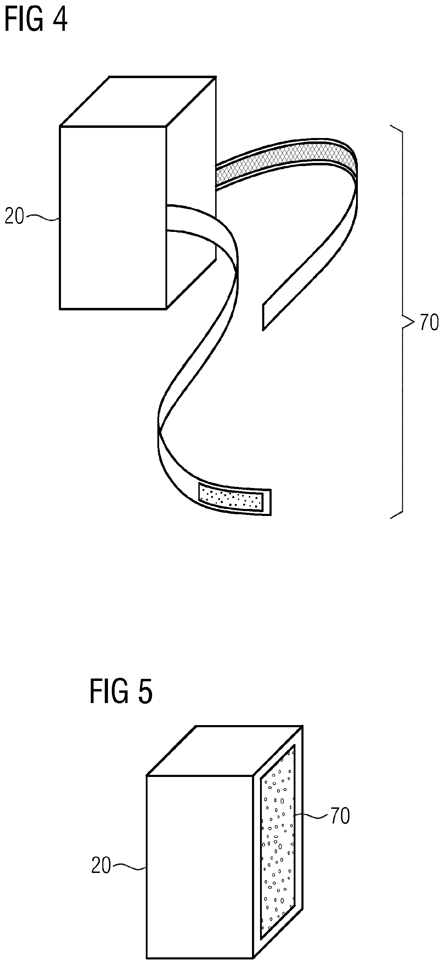

Alle hier gezeigten Beschleunigungssensoren kennzeichnen sich dadurch, dass sie einen Schnellverschluss 70 zum leichten Befestigen des Beschleunigungssensors an einem ersten medizinischen Gerät umfassen. In Figur 3 ist der Schnellverschluss 70 als längenverstellbarer Steckverschluss ausgebildet, sodass er leicht an die Größen bzw. Geometrie eines ersten medizinischen Geräts angepasst werden kann. In Figur 4 ist der Schnellverschluss 70 als Klettverschluss ausgebildet, wobei hier per sie die Länge variabel angepasst werden kann. In Figur 5 ist der Schnellverschluss 70 als flächiges Klebeelement ausgebildet, bspw. als doppelseitige Klebefolie, mit der der Beschleunigungssensor einfach auf eine glatte und flache Fläche eines ersten medizinischen Geräts 10, 11 befestigt werden kann. Weitere Alternativen für einen Schnellverschluss wären bspw. einfache Bänder oder Kabelbinder.

Die Ausführungsbeispiele der Figuren 3 und 4 sind darüber hinaus lösbar und wiederverschließbar ausgebildet. Die mit derartigen Schnellverschlüssen 70 versehenen Beschleunigungssensoren können auf einfache Weise neu bzw. umpositioniert werden. Insbesondere können sie derart auch von einem an ein anderes erstes medizinisches Gerät angebracht und dort wiederverwendet werden.

Wo noch nicht explizit geschehen, jedoch sinnvoll und im Sinne der Erfindung, können einzelne Ausführungsbeispiele, einzelne ihrer Teilaspekte oder Merkmale mit einander kombiniert bzw. ausgetauscht werden, ohne den Rahmen der hiesigen Erfindung zu verlassen. Mit Bezug zu einem Ausführungsbeispiel beschriebene Vorteile der Erfindung treffen ohne explizite Nennung, wo übertragbar, auch auf andere Ausführungsbeispiele zu.

## Patentansprüche

1. Medizinisches System (1) zur Kollisionsüberwachung in einer medizinischen Umgebung umfassend wenigstens ein erstes medizinisches Gerät (10, 11) und wenigstens einen Beschleunigungssensor (20, 21),
wobei das erste medizinische Gerät zur Ausführung eines medizinischen Arbeitsablaufes eine Zielposition (Z) in der medizinischen Umgebung einnimmt,
wobei der Beschleunigungssensor
- an dem ersten medizinischen Gerät angeordnet und
- ausgebildet ist, bei kollisionsbedingter Bewegung des ersten medizinischen Geräts aus seiner Zielposition heraus eine Beschleunigung zu erfassen und
- ein Warnsignal (W) an die medizinische Umgebung auszugeben,
wobei der Beschleunigungssensor ausgebildet ist, ein optisches und/oder akustisches Warnsignal auszugeben,
wobei das medizinische System ein zweites medizinisches Gerät (40, 50) umfasst, welches ausgebildet ist, in der medizinischen Umgebung eine teilautonome oder autonome Bewegung auszuführen, und
wobei das zweite medizinische Gerät ausgebildet ist, das optische und/oder akustische Warnsignal des wenigstens einen ersten Beschleunigungssensors zu erfassen und seine teilautonome oder autonome Bewegung zu stoppen.

2. Medizinisches System nach Anspruch 1, wobei der Beschleunigungssensor ausgebildet ist, eine Beschleunigung in einer beliebigen Raumrichtung zu erfassen.

3. Medizinisches System nach einem der vorherigen Ansprüche, wobei der Beschleunigungssensor ausgebildet ist, einen erfassten Wert der Beschleunigung mit einem vorab definierten Schwellwert zu vergleichen und nur bei Überschreiten des Schwellwerts ein Warnsignal auszugeben.

4. Medizinisches System nach einem der vorherigen Ansprüche, wobei der Beschleunigungssensor ausgebildet ist, eine Signalstärke des Warnsignals an einen erfassten Wert der Beschleunigung anzupassen.

5. Medizinisches System nach einem der Ansprüche 1 bis 4, wobei der Beschleunigungssensor einen Schnellverschluss (70) umfasst.

6. Medizinisches System nach Anspruch 5, wobei der Schnellverschluss lösbar und wiederverschließbar ausgebildet ist.

7. Medizinisches System nach einem der vorhergehenden Ansprüche, wobei das medizinische System eine Vielzahl von ersten medizinischen Geräten umfasst, wobei an jedem ersten medizinischen Gerät ein Beschleunigungssensor angeordnet ist.

8. Medizinisches System nach einem der vorherigen Ansprüche, wobei der wenigstens eine Beschleunigungssensor und das zweite medizinische Gerät über einen Datenkommunikationspfad miteinander in Kontakt stehen, der Beschleunigungssensor ausgebildet ist, ein Beschleunigungssignal an das zweite medizinische Gerät zu senden und das zweite medizinische Gerät ausgebildet ist, das Beschleunigungssignal zu erfassen.

## Claims

1. Medical system (1) for collision monitoring in a medical environment, comprising at least one first medical device (10, 11) and at least one acceleration sensor (20, 21),
wherein the first medical device assumes a target position (Z) in the medical environment in order to carry out a medical workflow,
wherein the acceleration sensor
- is arranged on the first medical device and
- is embodied to record an acceleration when the first medical device moves out of its target position due to a collision and
- to output a warning signal (W) to the medical environment,
wherein the acceleration sensor is embodied to output an optical and/or acoustic warning signal,
wherein the medical system comprises a second medical device (40, 50), which is embodied to carry out a semi-autonomous or autonomous movement in the medical environment, and
wherein the second medical device is embodied to record the optical and/or acoustic warning signal of the at least one first acceleration sensor and to stop its semi-autonomous or autonomous movement.

2. Medical system according to claim 1, wherein the acceleration sensor is embodied to record an acceleration in a given spatial direction.

3. Medical system according to one of the preceding claims, wherein the acceleration sensor is embodied to compare a recorded value of the acceleration with a previously defined threshold value and to only output a warning signal when the threshold value is exceeded.

4. Medical system according to one of the preceding claims, wherein the acceleration sensor is embodied to adapt a signal intensity of the warning signal to a recorded value of the acceleration.

5. Medical system according to one of claims 1 to 4, wherein the acceleration sensor comprises a quick-release closure (70) .

6. Medical system according to claim 5, wherein the quick-release closure is embodied as detachable and re-closable.

7. Medical system according to one of the preceding claims, wherein the medical system comprises a plurality of first medical devices, wherein an acceleration sensor is arranged on each first medical device.

8. Medical system according to one of the preceding claims, wherein the at least one acceleration sensor and the second medical device are in contact with one another via a data communication path, the acceleration sensor is embodied to transmit an acceleration signal to the second medical device and the second medical device is embodied to record the acceleration signal.

## Revendications

1. Système (1) médical de contrôle de collision dans un environnement médical comprenant au moins un premier appareil (10, 11) médical et au moins un capteur (20, 21) d'accélération,
dans lequel le premier appareil médical prend, pour effectuer une opération de travail médical, une position (Z) cible dans l'environnement médical,
dans lequel le capteur d'accélération
- est monté sur le premier appareil médical et
- est constitué pour, s'il se produit un déplacement, dû à une collision, du premier appareil médical, hors de sa position cible, en détecter une accélération et
- émettre un signal (W) d'alerte à l'environnement médical,
dans lequel le capteur d'accélération est constitué pour émettre un signal d'alerte optique et/ou acoustique,
dans lequel le système médical comprend un deuxième appareil (40, 50) médical, qui est constitué pour effectuer dans l'environnement médical un mouvement partiellement autonome ou autonome, et
dans lequel le deuxième appareil médical est constitué pour détecter le signal d'alerte optique et/ou acoustique du au moins un premier capteur d'accélération et arrêter son déplacement en partie autonome ou autonome.

2. Système médical suivant la revendication 1, dans lequel le capteur d'accélération est constitué pour détecter une accélération dans n'importe quelle direction de l'espace.

3. Système médical suivant l'une des revendications précédentes, dans lequel le capteur d'accélération est constitué pour comparer une valeur détectée de l'accélération à une valeur de seuil définie auparavant et pour n'émettre un signal d'alerte, que si la valeur de seuil est dépassée.

4. Système médical suivant l'une des revendications précédentes, dans lequel le capteur d'accélération est constitué pour adapter une intensité du signal d'alerte à une valeur détectée de l'accélération.

5. Système médical suivant l'une des revendications 1 à 4, dans lequel le capteur d'accélération comprend une fermeture (70) rapide.

6. Système médical suivant la revendication 5, dans lequel la fermeture rapide est constituée de manière à pouvoir être défaite et refermée.

7. Système médical suivant l'une des revendications précédentes, dans lequel le système médical comprend une pluralité de premiers appareils médicaux, dans lequel un capteur d'accélération est monté sur chaque premier appareil médical.

8. Système médical suivant l'une des revendications précédentes, dans lequel le au moins un capteur d'accélération et le deuxième appareil médical sont en contact l'un avec l'autre sur un chemin de communication de données, le capteur d'accélération est constitué pour envoyer un signal d'accélération au deuxième appareil médical et le deuxième appareil médical est constitué pour détecter le signal d'accélération.
